(19) Europäisches Patentamt
European Patent Office
Office européen des brevets

(11) **EP 1 140 841 B1**

(12) **FASCICULE DE BREVET EUROPEEN**

(45) Date de publication et mention de la délivrance du brevet:
**17.08.2005 Bulletin 2005/33**

(21) Numéro de dépôt: **99973409.8**

(22) Date de dépôt: **01.12.1999**

(51) Int Cl.[7]: **C07D 211/88**, C07C 235/78

(86) Numéro de dépôt international:
**PCT/FR1999/002971**

(87) Numéro de publication internationale:
**WO 2000/035879 (22.06.2000 Gazette 2000/25)**

(54) **DERIVES DE 3-PHENYL-2, 6-DIOXOPIPERIDIN-3-YL PROPIONAMIDE ET LEUR PROCEDE DE PREPARATION**

DERIVATE VON 3-PHENYL-2, 6-DIOXOPIPERIDIN-3-YL PROPIONAMIDE UND VERFAHREN ZUR DEREN HERSTELLUNG

3-PHENYL-2,6-DIOXOPIPERIDIN-3-YL PROPIONAMIDE DERIVATIVES AND METHOD FOR PREPARING SAME

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **18.12.1998 FR 9816088**

(43) Date de publication de la demande:
**10.10.2001 Bulletin 2001/41**

(73) Titulaire: **Sanofi-Aventis**
**75013 Paris (FR)**

(72) Inventeurs:
- **CASTRO, Bertrand**
  **34130 Saint Aunes (FR)**
- **MATTRAS, Hélène**
  **34570 Pignan (FR)**
- **PREVIERO, Aldo**
  **Chapelle sur Glane (CH)**

(74) Mandataire: **Thouret-Lemaitre, Elisabeth et al**
**Sanofi-Aventis**
**174 avenue de France**
**75013 Paris (FR)**

(56) Documents cités:
**EP-A- 0 673 928**
**WO-A-97/10211**
**WO-A-97/32852**
**WO-A-98/05640**
**WO-A-99/01451**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention a pour objet des dérivés de 3-(3-phényl-2,6-dioxopipéridin-3-yl)propionamide, leur procédé de préparation et leur utilisation.

L'acide 3-(3,4-dichlorophényl)-2,6-dioxopipéridine-3-propionique est décrit dans la demande de brevet WO 97/32852.

On a maintenant trouvé un nouveau composé ayant la configuration décrite par la formule :

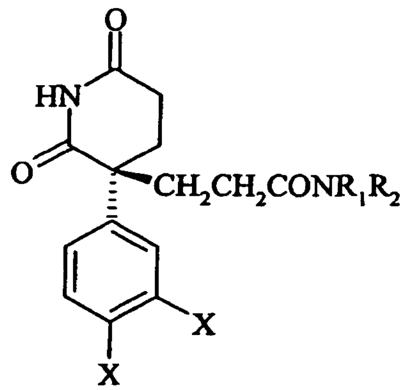

(II)

dans laquelle :

- X représente un atome de chlore ou un atome de fluor ;
- $R_1$ et $R_2$ ensemble constituent avec l'atome d'azote auquel ils sont liés un radical hétérocyclique choisi parmi l'azétidin-1-yle, le pyrrolidin-1-yle, la pipéridin-1-yle et la morpholin-4-yle.

lesdits radicaux hétérocycliques pouvant être mono ou disubstitués.

Comme substituants desdits radicaux hétérocycliques on peut citer des groupes semblables ou différents choisis parmi un alkyle en $C_1$-$C_4$, un phényle, un benzyle, un amino, un ($C_1$-$C_4$)alkylamino et un di ($C_1$-$C_4$)alkylamino.

On préfère les composés de formule (II) dans lesquelles $NR_1R_2$ représente un groupe morpholino ou pipéridino. On préfère également les composés de formule (II) dans lesquelles $NR_1R_2$ représente un groupe 4-méthylamino-4-phénylpipéridin-1-yle.

Les composés de formule (II) sont des intermédiaires utiles pour la préparation des composés de formule :

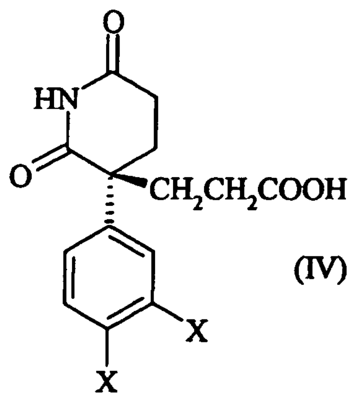

(IV)

dans lesquelles X est tel que défini ci-dessus pour (II).

Il apparaît dans la demande de brevet WO 97/03852 que les composés de formule (IV) sont utiles pour la préparation de composés antagonistes des neurokinines tels que celui décrit dans la publication de X. Emonds-Alt et al., Life Sci., 1995, <u>56</u> (1), 27-32, à savoir le (S)-N-[1-[3-{1-benzoyl-3-(3,4-dichlorophényl)pipéridin-3-yl} propyl]-4-phénylpipéridin-4-yl]-N-méthylacétamide ou SR 142801 dont la dénomination commune internationale est osanétant. Ce composé est un $NK_3$ antagoniste.

La présente invention a également pour objet un procédé pour la préparation d'un composé de formule (II)

caractérisé en ce que l'on cyclise un composé de formule :

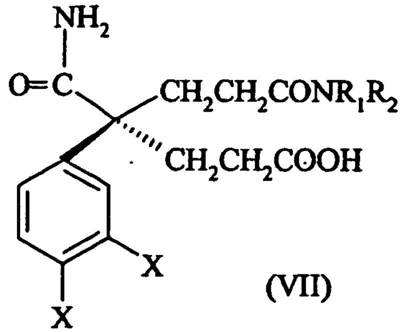

(VII)

dans laquelle X est tel que défini ci-dessus pour (II), par élimination d'eau, pour obtenir un composé de formule :

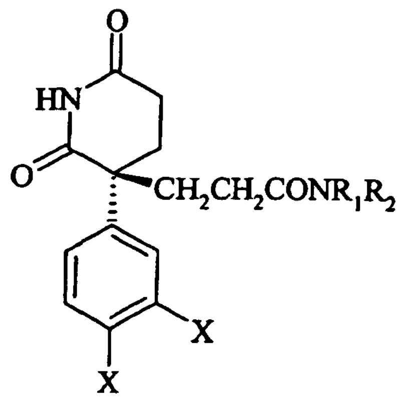

(II)

Le composé de formule (VII) peut être obtenu par action d'une amine de formule $HNR_1R_2$ dans laquelle $R_1$ et $R_2$ sont tels que définis ci-dessus pour (II) sur un composé de formule :

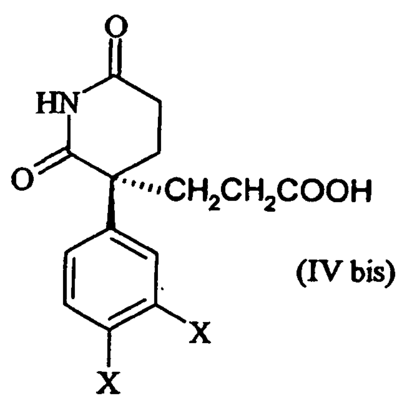

(IV bis)

La préparation du composé de formule (VII) est réalisée dans l'eau, dans un solvant alcoolique, dans un solvant éthéré, dans un solvant chloré ou dans un solvant aromatique, à une température comprise entre la température ambiante et la température de reflux du solvant. Préférentiellement, on utilise l'eau ou le méthanol.

La cyclisation du composé de formule (VII) par élimination d'une molécule d'eau se fait après activation du groupe carboxyle du composé de formule (VII) par formation d'un anhydride mixte. Pour cela, on utilise un anhydride en excès, par exemple l'anhydride acétique ou l'anhydride méthanesulfonique, à une température comprise entre la

température ambiante et 100°C.

Les composés de formule (VII) sont nouveaux et font partie de la présente invention.

Les composés de formule (IVbis) sont décrits dans la demande de brevet WO 97/32852.

Selon la présente invention, on a trouvé, en utilisant comme produit de départ un énantiomère pur de formule (IVbis), que la formation du dérivé d'acide hexanoique de formule (VII) se fait avec rétention de configuration, et que, ensuite, la cyclisation se fait avec inversion de configuration.

Selon la présente invention, on peut préparer le composé de formule :

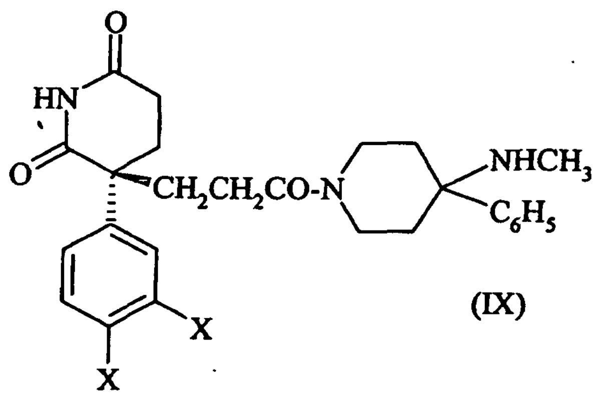

(IX)

en utilisant un autre procédé caractérisé en ce que l'on fait agir la 4-méthylamino-4-phénylpipéridine sur un composé de formule :

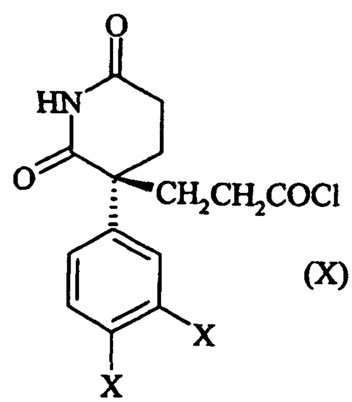

(X)

Le composé de formule (X) peut être préparé à partir de l'acide de formule :

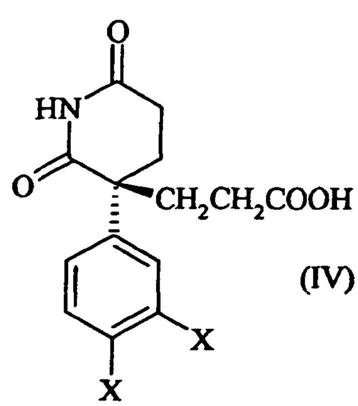

(IV)

Le chlorure d'acide est préparé de façon connue, par exemple par action du chlorure de thionyle en l'absence

de solvant ou dans un solvant anhydre tel que le toluène, le diméthylformamide ou le dichlorométhane ou encore dans un mélange de ces solvants.

L'action de la 4-méthylamino-4-phénylpipéridine est réalisée dans un solvant anhydre, en milieu basique, par exemple en présence de triéthylamine.

La 4-méthylamino-4-phénylpipéridine est préparée selon Biorg. Med. Chem. Letters, 1996, 4 (19), 2307-2310.

Selon la présente invention, le composé de formule (II) permet d'obtenir par acidolyse l'acide de formule :

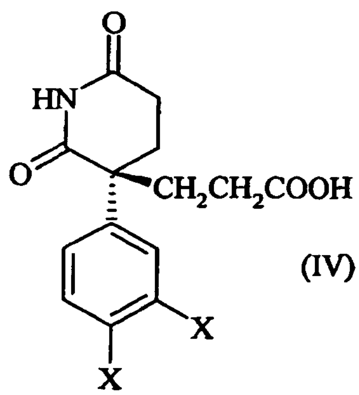

(IV)

L'acidolyse est réalisée de façon connue de l'homme de l'art, par exemple par un acide carboxylique en $C_1$-$C_4$.

Ainsi, la présente invention concerne un procédé de préparation d'un composé de formule (IV) par hydrolyse d'un composé de formule (II).

Selon un aspect ultérieur de la présente invention, par réduction d'un composé de formule (IX) dans laquelle X = Cl, on prépare un composé de formule :

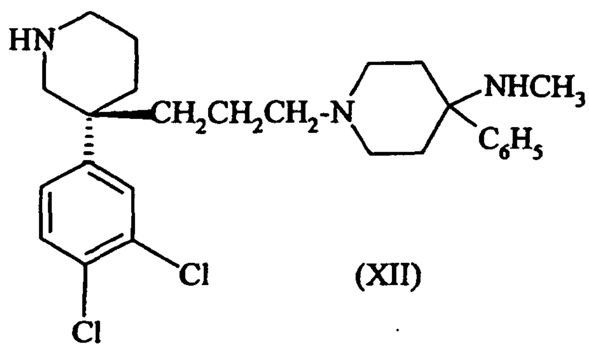

(XII)

La réduction est effectuée par des moyens connus de l'homme de l'art. Les agents de réduction utilisés sont des complexes du borane tels que par exemple le borane-tétrahydrofurane ou le borane-diméthylsulfure ou encore un hydrure mixte alcalin tel que l'hydrure de lithium aluminium ou l'hydrure de sodium bis (2-méthoxyéthoxy)aluminium en solution dans le toluène (Red-Al®), le complexe de borane-tétrahydrofurane étant préféré.

La réduction avec le borane est conduite dans un solvant de préférence aprotique tel que le tétrahydrofurane à la température de reflux du solvant. En général, après 1 à 6 heures de chauffage, la réduction est complète et la pipéridine 3,3-disubstituée est isolée, selon les méthodes conventionnelles, en détruisant d'abord l'excès de borane avec du méthanol. La base libre peut être isolée par évaporation du solvant, puis on reprend le résidu par de l'eau, on acidifie avec de l'acide chlorhydrique, on traite avec une base, de préférence l'hydroxyde de sodium, et on extrait avec un solvant.

La présente invention concerne un procédé de préparation d'un composé de formule (XII) par réduction d'un composé de formule (IX).

Dans les exemples et dans la description, les abréviations suivantes sont utilisées.

TA : température ambiante
Me : méthyle
THF : tétrahydrofurane
DMF : diméthylformamide

L-PheOMe : ester méthylique de la L-phénylalanine
BOP (Castro's reagent) : Hexafluorophosphate de benzotriazol-1-yloxytris (diméthylamino)phosphonium
MIBK : méthylisobutylcétone.

Les spectres de RMN (résonnance magnétique nucléaire) sont enregistrés à 250 ou 300 MHz.
Pour analyser les produits, on utilise la chromatographie liquide haute pression sur C18 (HPLC-C18) dans les conditions suivantes :

10 à 15 µmoles de produit dissoutes dans 50 µl de méthanol sont diluées avec 150 µl d'éluant, et on injecte 20 µl sur Colonne Alltima® C18 5 µ (longueur = 250 mm, diamètre intérieur = 4,6 mm) en éluant par un mélange isocratique A/B : 32/68, débit 1ml/min ; A : tampon aqueux pH 2,6 ; B : méthanol ; on détecte les composés à 280 mm.

On utilise également la HPLC-C18 chirale dans les conditions analytiques suivantes :

2 à 3 µmoles de produit sont dissoutes dans un mélange de DMF (100 µl), triéthylamine (15 µl), avec soit (S) méthylbenzylamine (15 µl), soit L-PheOMe (15 mg). On ajoute 10 mg de BOP (Castro's reagent), et on laisse 30 minutes à 50°C. Le mélange réactionnel est soumis à une extraction acide par acétate d'éthyle/HCl 1N : 1ml/1ml. 100 µl de phase organique sont alors évaporés puis soit repris par 250 µl d'éluant, soit repris par 50 µl de méthanol puis dilués par 150 µl d'éluant. On injecte 20 µl de cette solution. On opère sur des colonnes de longueur 250 mm et de diamètre intérieur 4,6 mm en éluant par un mélange hexane/isopropanol : 70/30, débit 1 ml/min. On détecte les composés à 280 nm.

Les autres conditions analytiques varient selon les composés analysés.

Préparation 1

Acide 4-carbamoyl-4-(3,4-dichlorophényl)-6-(pipéridin-1-yl)carbonyl hexanoïque.

(VI) : X = Cl, $NR_1R_2$ = 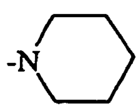

0,3 g d'acide 3-[3-(3,4-dichlorophényl)-2,6-dioxo pipéridin-3-yl]propionique, racémique, 1 ml de méthanol et 0,5 ml de pipéridine sont placés à 60°C pendant15 heures dans un ballon bouché. Le milieu réactionnel est dilué avec 10 ml d'eau et acidifié par HCl 1N à pH 2-3. Les cristaux blancs formés sont filtrés, essorés puis séchés pour donner 0,3 g du composé attendu.
RMN $^1$H (DMSO) : 1,36 à 1,56 ppm (3 $CH_2$, m) ; 2, 04 à 2, 50 ppm (4 $CH_2$, m) ; 3,3 ppm (2 $CH_2$, m) ; 7,25 et 7,6 ppm (2H aromatiques, d) ; 7,45 ppm (1H aromatique, d) ; 10,98 ppm (COOH, s).
L'analyse par HPLC-C18 montre un pic à Tr = 8,2 minutes.
L'analyse chirale HPLC-C18, après couplage avec L-PheOMe, montre la formation de 2 pics de surface identique : Tr = 7,9 minutes et Tr = 8,8 minutes.
Le composé obtenu est donc sous forme racémique.

Préparation 2

Acide 4-carbamoyl-4-(3,4-dichlorophényl)-6-(pipéridin-1-yl)carbonylhexanoïque, de configuration S.

(VII) : X = Cl, $NR_1R_2$ = 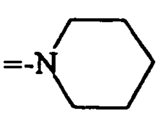

0,3 g d'acide 3-[3-(3,4-dichlorophényl)-2,6-dioxo pipéridin-3-yl]propionique de configuration S sont traités par

la pipéridine, comme décrit à la Préparation 1 pour donner 0,3 g du composé attendu.

L'analyse par HPLC-C18 montre un pic à Tr = 8,2 minutes.

L'analyse chirale par HPLC-C18 après couplage avec L-PheOMe, montre un pic à Tr = 8,8 minutes. On constate que la pureté optique est identique à celle du produit de départ.

-6,4° (c = 1 ; $CH_3OH$/NaOH 1N : 9,7/0,3)

Préparation 2 bis

Acide 4-carbamoyl-4-(3,4-dichlorophényl)-6-(pipéridin-1-yl)carbonyl hexanoïque, de configuration R.

(VII) : X = Cl, $NR_1R_2$ =-N 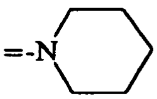

On opère comme à la préparation 2 en utilisant comme produit de départ l'acide 3-[3-(3,4-dichlorophényl)-2,6-dioxopipéridin-3-yl]propionique de configuration R.

L'analyse chirale après couplage avec L-PheOMe, montre un pic à Tr = 7,9 minutes.

EXEMPLE 1

3-(3,4-dichlorophényl)-3-[(pipéridin-1-yl)-3-oxopropyl]-pipéridine-2,6-dione, de configuration R.

(II) : X = Cl, $NR_1R_2$ = -N 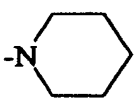

0,2 g du composé obtenu à la Préparation 2 et 0,8 ml d'anhydride acétique sont placés dans un tube fermé à 95-100°C pendant 30 minutes. Après refroidissement à TA, on ajoute 10 ml d'eau distillée et on laisse sous agitation 30 à 40 minutes. On extrait par 10 ml d'acétate d'éthyle puis la phase acétate d'éthyle est séchée puis évaporée sous pression réduite. On obtient 0,18 g du composé attendu. L'analyse par HPLC-C18 montre un seul pic à Tr = 14 minutes.

EXEMPLE 1 bis

3-(3,4-dichlorophényl)-3-[(pipéridin-1-yl)-3-oxopropyl]-pipéridine-2,6-dione, de configuration R.

On opère comme à l'exemple 1, à partir du composé obtenu à la préparation 2 bis.

L'analyse par HPLC-C18 du composé obtenu montre un seul pic à Tr = 14 minutes.

EXEMPLE 2

Acide 3-[3-(3,4-dichlorophényl)-2,6-dioxopipéridin-3-yl]propionique, de configuration S.

(III) : X = Cl.

Le composé obtenu à l'EXEMPLE 1 est repris dans 0,5 ml d'acide propionique et placé dans un tube scellé

sous vide. Après 18 heures à 145°C, le mélange réactionnel et dilué par 10 ml d'HCl 1N. Après une journée, le produit qui a cristallisé est filtré, essoré et séché. On obtient 100 mg du composé attendu.

L'analyse HPLC-C18 montre un pic à Tr = 6,2 minutes.

L'analyse chirale après couplage avec la (S)-méthylbenzylamine montre un pic à 7,13 minutes.

EXEMPLE 2 bis

Acide 3-[3-(3,4-dichlorophényl)-2,6-dioxopipéridin-3-yl]propionique, de configuration S.

On opère comme à l'exemple 2 à partir du composé obtenu à l'exemple 1 bis.

L'analyse par HPLC-C18 du composé obtenu montre un pic à Tr = 6,2 minutes.

L'analyse chirale après couplage avec la (S)-méthylbenzylamine montre un pic à 9,13 minutes.

EXEMPLE 3

3-(3,4-Dichlorophényl)-3-[3-(4-méthylamino-4-phénylpipéridin-1-yl)-3-oxopropyl]-pipéridine-2,6-dione, de configuration S.

(IX) : X = Cl

Chlorure de l'acide 3-[3-(3,4-dichlorophényl)-2,6-dioxopipéridin-3-yl]propionique, de configuration S.

Sous atmosphère d'azote, on mélange 20 g d'acide 3-[3-(3,9-dichlorophényl)-2,6-dioxopipéridin-3-yl]propionique, 200 ml de toluère et 0,5 ml de DMF. On chauffe à 60°C et on introduit goutte à goutte 14,9 g de chlorure de thionyle et 40 ml de toluène. On maintient l'agitation pendant 5 heures à 60°C. On évapore le toluène et l'excès de chlorure de thionyle puis on sèche le milieu réactionnel à l'étuve (40°C) une nuit.

Le produit obtenu est utilisé tel quel à l'étape suivante.

B) 3-(3,4-Dichlorophényl)-3-[3-(4-méthylamino-4-phénylpipéridin-1-yl)-3-oxopropyl]-pipéridine-2,6-dione, de configuration S.

Sous atmosphère d'azote, on introduit 11,5 g de 4-méthylamino-4-phénylpipéridine, en solution dans 100 ml de THF et 8,52 ml de triéthylamine puis on refroidit le milieu réactionnel à 0°C ± 5°C avec un bain de glace. On ajoute goutte à goutte le chlorure d'acide préparé à l'étape précédente dans 150 ml de THF et laisse sous agitation pendant une heure. Après évaporation du THF, on lave avec 100 ml d'eau et 100 ml de dichlorométhane. La phase organique est décantée puis lavée 3 fois par 100 ml d'eau ; on évapore le dichlorométhane puis on sèche à l'étuve à 40°C et l'on obtient 32,1 g du composé attendu. 29,1 g de ce composé sont recristallisés à chaud dans MIBK pour donner 17 g du composé pur. Par ailleurs, les eaux-mères de la recristallisation sont filtrées sur silice pour donner 5,2 g supplémentaire de composé pur.

RMN $^{1}H$ ($CDCl_3$) : 1,65 à 2,69 ppm (16 H, m) ; 3,25 à 3, 98 ppm (4 H, m) ; 7,06 à 7, 46 ppm (8H aromatiques et CO-NH-CO).

$\alpha_D^{25}$ = + 77,8° (c = 1, méthanol)

## Revendications

1. Un composé ayant la configuration décrite par la formule :

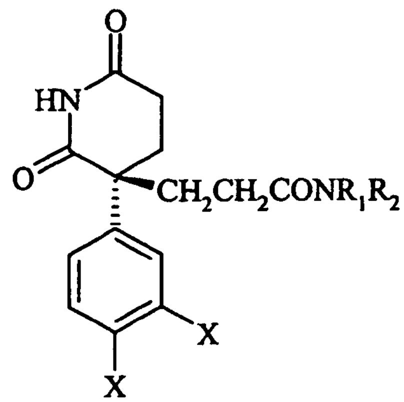

(II)

dans laquelle :

- X représente un atome de chlore ou un atome de fluor.
- $R_1$ et $R_2$ ensemble constituent avec l'atome d'azote auquel ils sont liés un radical hétérocyclique choisi parmi l'azétidin-1-yle, le pyrrolidin-1-yle, la pipéridin-1-yle et la morpholin-4-yle.

lesdits radicaux hétérocycliques pouvant être mono ou disubstitués par un ou des groupes semblables ou différents choisis parmi un alkyle en $C_1$-$C_4$, un phényle, un benzyle, un amino, un ($C_1$-$C_4$)alkylamino et un di ($C_1$-$C_4$)alkylamino.

**2.** Un composé selon la revendication 1 de formule :

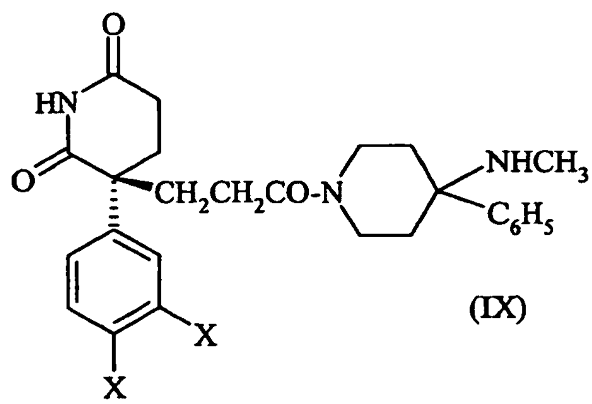

(IX)

**3.** Un procédé de préparation d'un composé de formule (II) selon la revendication 1 **caractérisé en ce que** l'on cyclise un composé de formule :

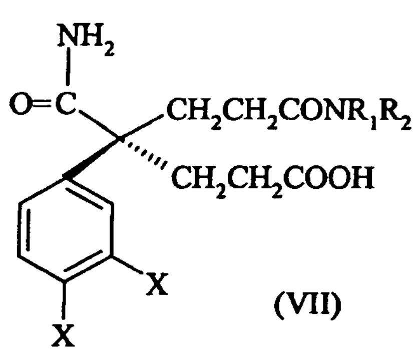

(VII)

dans laquelle X est tel que défini pour (II) à la revendication 1, par élimination d'eau, pour obtenir un composé de formule :

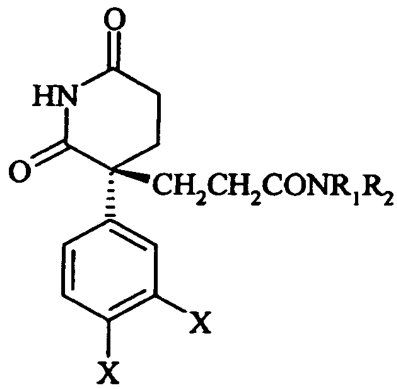

(II)

**4.** Un procédé selon la revendication 3 **caractérisé en ce que** le composé de formule (VII) est obtenu par action d'une amine de formule $HNR_1R_2$ dans laquelle $R_1$ et $R_2$ sont tels que définis pour (II) à la revendication 1 sur un composé de formule :

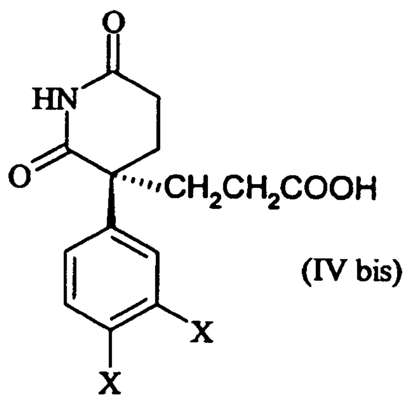

(IV bis)

**5.** Un procédé de préparation d'un composé de formule (IX) selon la revendication 2 **caractérisé en ce que** l'on fait agir la 4-méthylamino-4-phénylpipéridine sur un composé de formule :

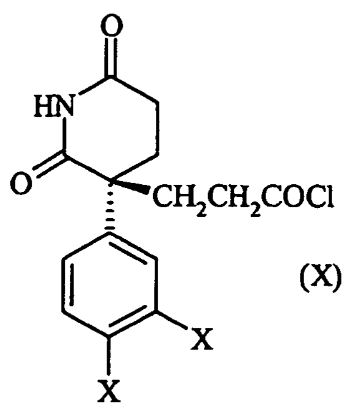

(X)

pour obtenir un composé de formule :

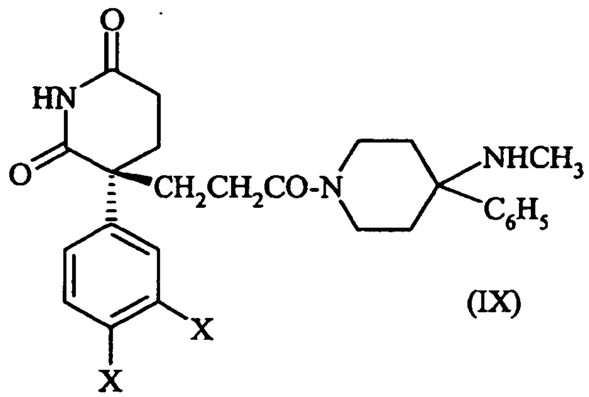

(IX)

6. Un procédé selon la revendication 5 **caractérisé en ce que** le composé de formule (X) est préparé à partir d'un acide de formule :

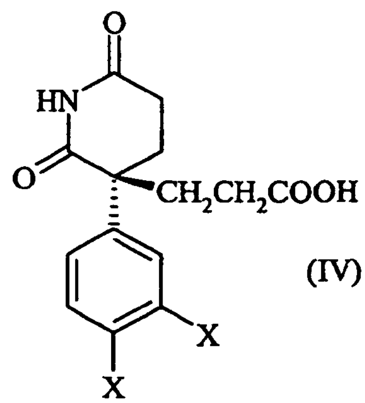

(IV)

7. Un composé de formule :

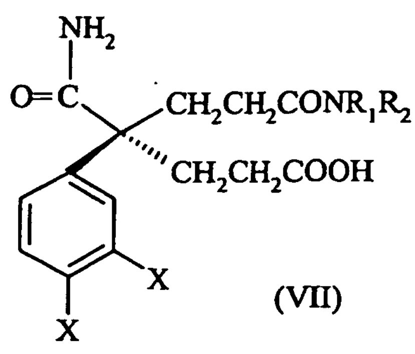

(VII)

8. Utilisation d'un composé de formule :

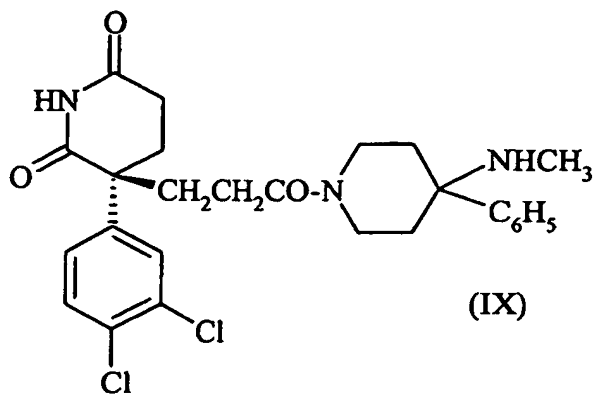

(IX)

pour la préparation d'un composé de formule :

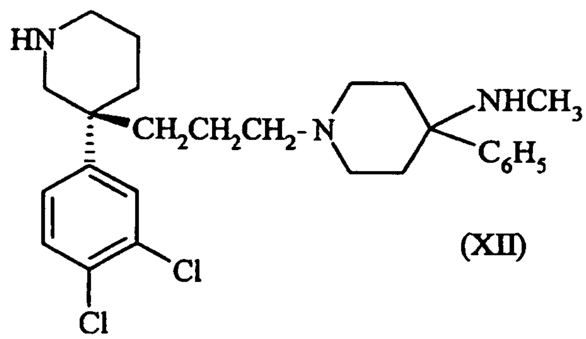

(XII)

**Claims**

1. Compound having the configuration described by the formula:

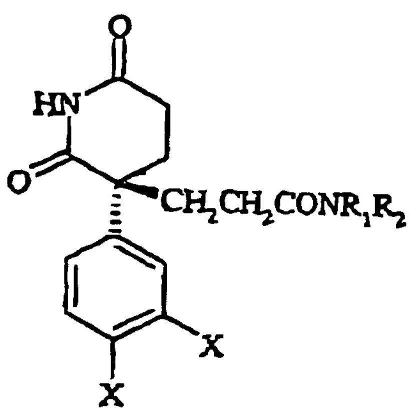

(II)

in which:

- X represents a chlorine atom or a fluorine atom;
- $R_1$ and $R_2$ together constitute with the nitrogen atom to which they are attached a heterocyclic radical chosen from azetidin-1-yl, pyrrolidin-1-yl, piperidin-1-yl and morpholin-4-yl,

it being possible for said heterocyclic radicals to be mono- or disubstituted with one or more groups, which are similar or different, chosen from a $C_1$-$C_4$ alkyl, a phenyl, a benzyl, an amino, a ($C_1$-$C_4$)alkylamino and a di ($C_1$-$C_4$) alkylamino.

**2.** Compound according to Claim 1 of formula:

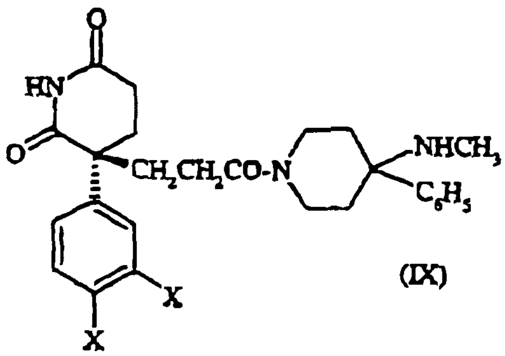

(IX)

**3.** Method of preparing a compound of formula (II) according to Claim 1, **characterized in that** a compound of formula:

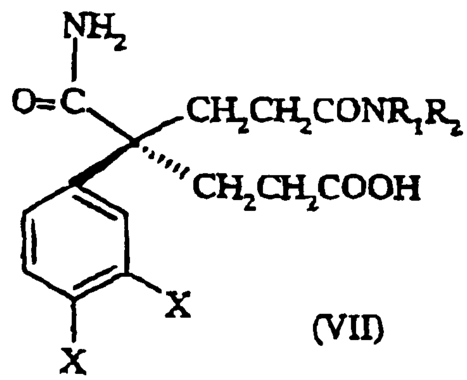

(VII)

in which X is as defined for (II) in Claim 1, is cyclized by elimination of water, to give a compound of formula:

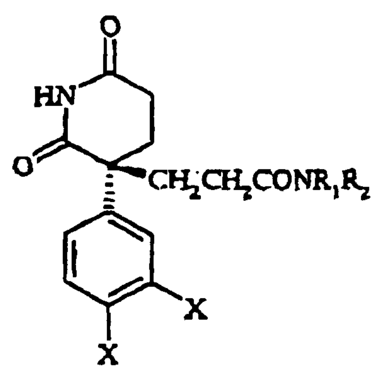

(II)

**4.** Method according to Claim 3, **characterized in that** the compound of formula (VII) is obtained by the action of an amine of formula $HNR_1R_2$ in which $R_1$ and $R_2$ are as defined for (II) in Claim 1 on a compound of formula:

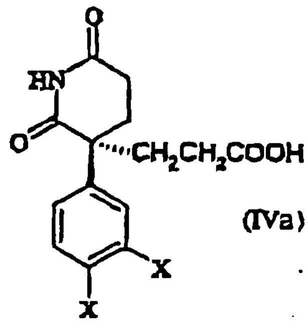

(IVa)

.

5. Method for preparing a compound of formula (IX) according to Claim 2, **characterized in that** 4-methylamino-4-phenylpiperidine is reacted with a compound of formula:

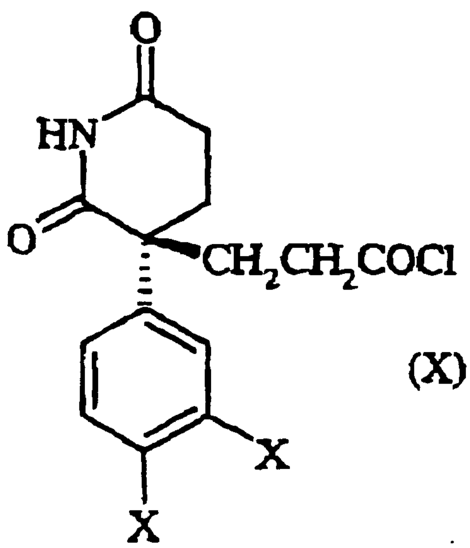

(X)

.

to give a compound of formula:

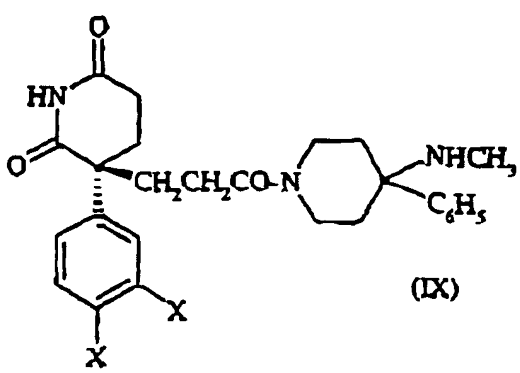

(IX)

6. Method according to Claim 5, **characterized in that** the compound of formula (X) is prepared from an acid of formula:

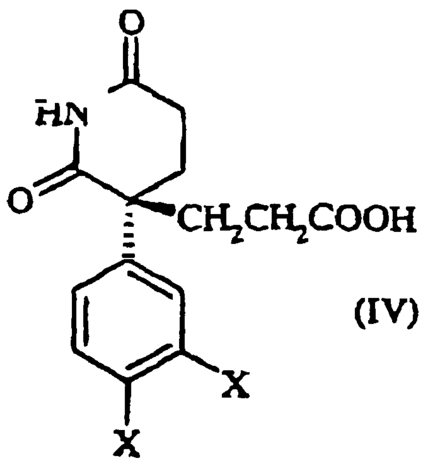

(IV)

7. Compound of formula:

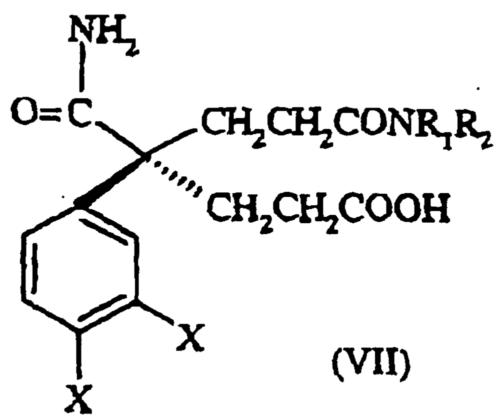

(VII)

8. Use of a compound of formula:

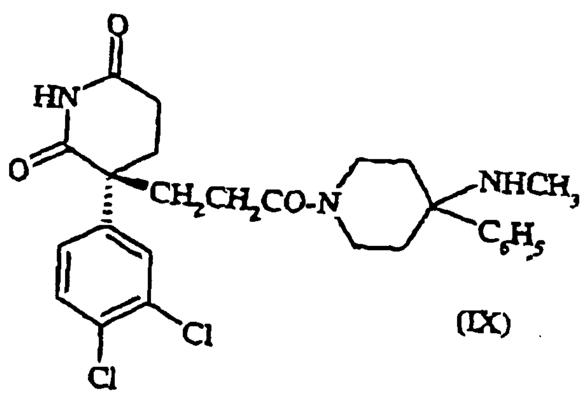

(IX)

for the preparation of a compound of formula:

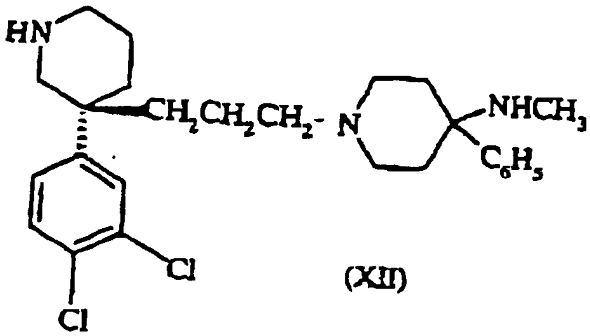

(XII)

**Patentansprüche**

1. Verbindung mit der durch die Formel

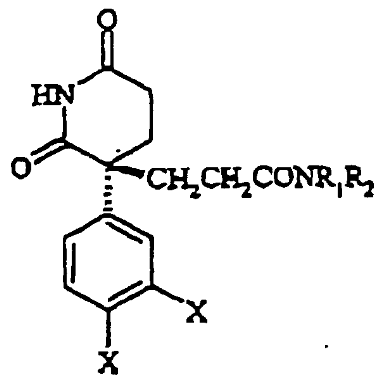

(II)

beschriebenen Konfiguration, in der

- X ein Chloratom oder ein Fluoratom darstellt,
- $R_1$ und $R_2$ zusammen mit dem Stickstoffatom, an das sie gebunden sind, einen heterocyclischen Rest bilden, ausgewählt unter Azetidin-1-yl, Pyrrolidin-1-yl, Piperidin-1-yl und Morpholin-4-yl,

wobei die genannten heterocyclischen Reste mono- oder disubstituiert sein können durch eine oder mehrere gleiche oder verschiedene Gruppen, ausgewählt unter einem ($C_1$-$C_4$)-Alkyl, einem Phenyl, einem Benzyl, einem Amino, einem ($C_1$-$C_4$)-Alkylamino und einem Di($C_1$-$C_4$)-alkylamino.

2. Verbindung nach Anspruch 1 der Formel (IX)

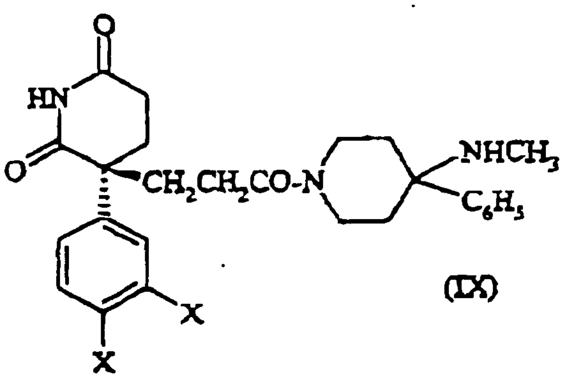

(IX)

3. Verfahren zur Herstellung einer Verbindung der Formel (II) nach Anspruch 1, **dadurch gekennzeichnet, daß** man eine Verbindung der Formel (VII)

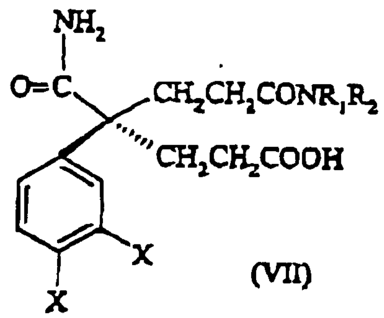

(VII)

in der X wie bei (II) in Anspruch 1 definiert ist, durch Entfernung von Wasser cyclisiert, um eine Verbindung der Formel (II)

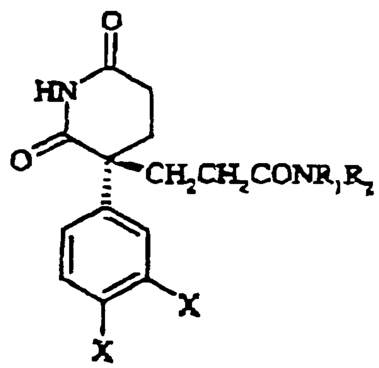

(II)

zu erhalten.

4. Verfahren nach Anspruch 3, **dadurch gekennzeichnet, daß** die Verbindung der Formel (VII) erhalten wird durch Einwirkung eines Amins der Formel $HNR_1R_2$, in der $R_1$ und $R_2$ wie bei (II) in Anspruch 1 definiert sind, auf eine Verbindung der Formel (IV bis)

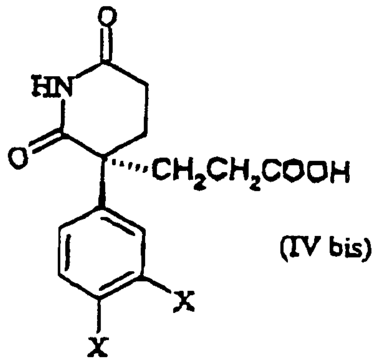

(IV bis)

**5.** Verfahren zur Herstellung einer Verbindung der Formel (IX) nach Anspruch 2, **dadurch gekennzeichnet, daß** man 4-Methylamino-4-phenylpiperidin mit einer Verbindung der Formel (X)

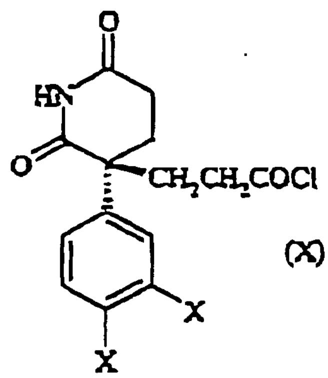

(X)

zur Reaktion bringt, um eine Verbindung der Formel (IX)

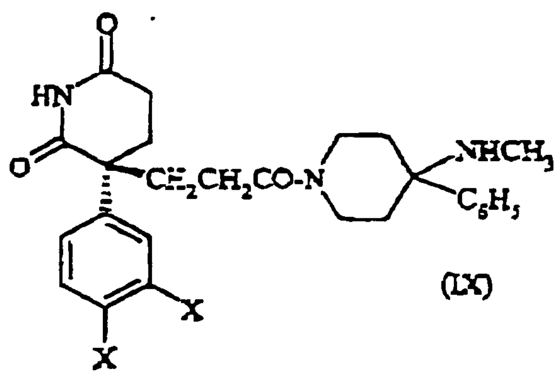

(IX)

zu erhalten.

**6.** verfahren nach Anspruch 5, **dadurch gekennzeichnet, daß** die Verbindung der Formel (X) ausgehend von einer Säure der Formel (IV)

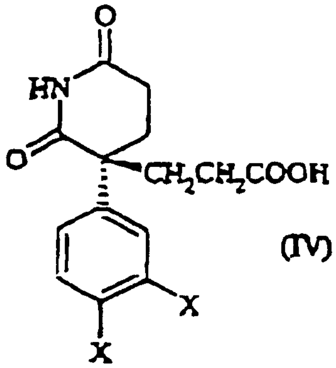

(IV)

hergestellt wird.

**7.** Verbindung der Formel (VII)

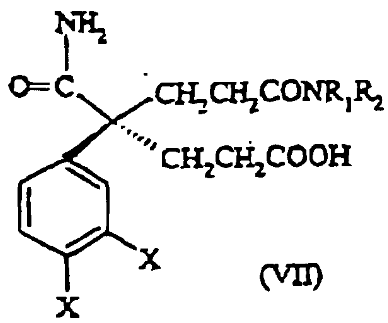

(VII)

**8.** Verwendung einer Verbindung der Formel (IX)

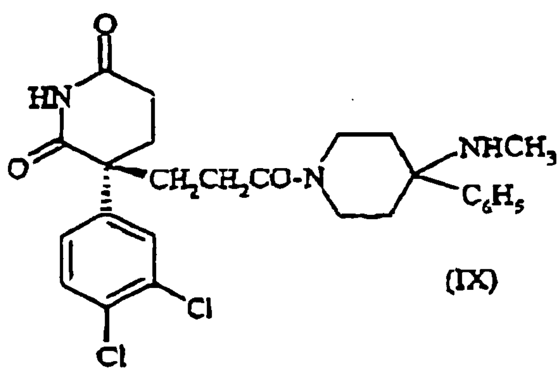

(IX)

zur Herstellung einer Verbindung der Formel (XII)

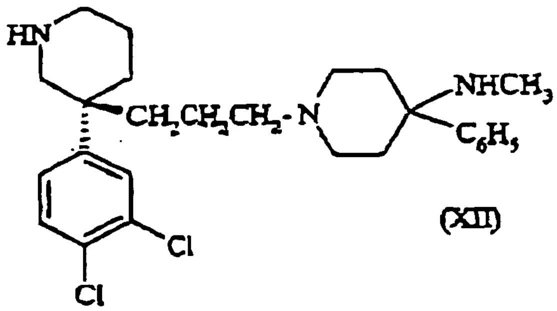

(XII)